# EUROPEAN PATENT APPLICATION

(11) **EP 1 559 688 A1**
(43) Date of publication of application: **03.08.2005**
(21) Application number: 05447008.3
(22) Date of filing: 28.01.2005
(51) Int. Cl.: C02F 11/04, C02F 3/30, C02F 11/02

(54) **A method for the degradation of organic sludge**

(30) Priority: 29.01.2004 NL 1025346
(71) Applicant: Seghers Keppel Technology Group, 2830 Willebroek (BE)
(72) Inventor: Pipyn, Pascal, 2830 Willebroek (BE)
(74) Representative: Luys, Marie-José

(57) **Abstract**

The present invention relates to a method for the degradation of sludge and/or solid components of organic origin present in waste water, according to which the sludge is successively subjected to an anaerobic and aerobic biologic fermentation process, wherein the method comprises the following steps: a) the sludge is subjected to an anaerobic biologic fermentation process (1), in which a first biomass with a first interstitial liquid phase is formed and methane gas is produced; b) the thus obtained first biomass and first interstitial liquid phase are transported to an aerobic fermentation tank (2) and subjected to an aerobic fermentation in the presence of an oxygen containing gas, during which the temperature is maintained between 25 and 70°C and a second biomass with an interstitial second effluent is formed; c) after which the thus formed second biomass and interstitial effluent are conducted towards a separator (4) for separating the sludge into purified effluent and a second biomass. In a preferred embodiment the sludge may be subjected to a physical disintegration and/or chemical and/or thermal hydrolysis before being re-circulated.

## Description

The present invention relates to a method for the degradation of organic sludge and/or solid components of organic origin present in waste water, according to which the sludge is successively subjected to an anaerobic and aerobic biologic fermentation process as disclosed in the preamble of the first claim.

In the past a wide variety of processes and waste water purification devices have been developed for the treatment of waste water containing organic impurities, which originated from industrial activities as well as from urban agglomerations. These processes were concentrated to the aspect of the purification of waste water. The problem that in the course of the purification of waste water, sludge is generated as a waste product, had a lower priority and was only partly solved. Up to now, usually the sludge was dumped, However, an ever increasing accumulation of sludge is unwanted. Therefore, it is the aim of the present invention to provide a method with which sludge generated in other processes can be disintegrated to a large extent, with the aim of reducing the amount of accumulating sludge to a minimum.

With respect to sludge the following classes may be distinguished:
- Primary sludge which is produced in primary settling tanks. This sludge mainly contains organic residues and widely varying types of waste, such as for example organic raw materials, food residues etc
- Secondary sludge, which is produced in biologic water purification installations and which mainly consists of micro-organisms, the so-called biomass. Secondary sludge may contain several types of micro-organisms, such as for example, bacteria, fungi, yeasts, protozoa, nematodes, algae etc.

JP2001286885 in the name of Hitachi Kiden Kogyo KK discloses the so-called Pho-strip process for removing phosphor from waste water. Thereto, the sludge is subjected to a so-called solubilising process, whereby the sludge is temporarily conserved in the absence of oxygen in such a way that the micro-organisms release the phosphor that has accumulated in them, in a concentrated form. The thus obtained sludge, purified from phosphor, is dumped. JP2001286885 teaches that phosphor release may be accelerated by heating the sludge.

JP04156999 in the name of Meidensha Corp disclose an active sludge system for the purification of waste water, which is preceded by a phosphor removal step. Phosphor removal is carried out in the absence of oxygen. It is alleged that an accelerated oxygen removal may be obtained by the addition of an amount of nitrifying sludge to the sludge.

JP60041594 in the name of Ebara Infilco discloses an active sludge system for removing phosphor from waste water. The phosphor which is released in the course of the anaerobic thickening is recovered by the sludge by aerating the sludge, after having been thickened, but before water removal.

EP-A-543.457 in the name of TAUW Infra Consult, discloses a three-step active sludge system with the accent to the removal of mainly phosphor and nitrogen from waste water, and to a lesser extent BOD and COD. According to this method in a first highly loaded step, phosphor, BOD and COD are partly removed, in a second step a post-nitrification is carried out, which is followed by a post-denitrification while adding an external carbon source to further remove BOD and COD. The sludge remaining after the first step is subjected to a short fermentation process in anaerobic conditions to form fatty acids as a source of nutrients for the micro-organisms in the post-denitrification.

GB-A-1.438.697 in the name of Stamicarbon relates to a method for removing nitrogen compounds from waste water, according to which the waste water is subjected to an anaerobic denitrification in a first step, the effluent of the denitrification is subjected to an aerobic nitrification and part of the effluent is recycled to the denitrification. The sludge containing remainder is partly recycled to the denitrification, the remainder is dumped.

The above described patent publications all relate to a method for the purification of waste water, wherein an excess of sludge is produced as waste product. However, dumping of the sludge is becoming a problem.

From US-A-5.141.646 in the name of Rozich a method for the treatment of sludge and/or organic waste is known, comprising the following steps:
1. sludge containing approximately 4-8 % of solid components, is introduced into a milling device and conducted to a mixer, where the sludge is mixed.
2. the mixed sludge is conducted to an autothermal anaerobic fermentation reactor (AAD), for the purpose of withdrawing energy from the waste by recuperation of the methane formed in the course of the anaerobic fermentation. If so desired, water and/or nutrients can be supplied to the AAD reactor. If so desired, the excess of biomass present in the AAD reactor, can be removed. However this requires an additional process step.
3. the biomass which has settled in the AAD is conducted to a hydrolysis unit and is hydrolysed under the influence of strong acid or base, and recycled to the mixer if so desired. Usually 1 part of acid is used per 10 parts of solids.
4. the supernatant liquid of the AAD is conducted to an ATAD reactor (autothermal aerobic fermentation device). The waste is subjected to a thermofilic aerobic fermentation process at a temperature of approximately 50-70°C in the presence of oxygen. To permit sedimentation of the biomass formed therein, the ATAD reactor is for example interrupted once a day. Thereafter, part of the biomass is removed and conducted to the hydrolysis unit for hydrolysis, after which the hydrolysed biomass is recycled to the mixer and is mixed with the introduced waste. However, hardly any sedimentation occurs with the biomass.
5. the supernatant liquid is removed and can be subjected to a further treatment. It is for example possible to transport the supernatant liquid to an extraction device where the nutrients are extracted from the liquid, or the purified waste water is returned to the process in which the waste water was generated.

The method disclosed in US-A-5.141.646 however presents the disadvantage that the excess of biomass formed in the AAD reactor is processed by subjecting the biomass to a hydrolysis with chemicals. This involves a significant consumption of chemicals. The method disclosed in US-A-5.141.646 however presents the disadvantage that aerobic fermentation is carried out in an autothermal manner, which in practise corresponds to a temperature of below 25°C. However, at this temperature the autothermal fermentation proceeds very slow, in particular the fermentation of primary sludge.

NL-A-9301151 solves the problem of providing a method for the treatment of industrial, toxic waste, with a reduced production of sludge. Thereto, the sludge is subjected to an anaerobic fermentation in the AAD reactor, whereby a first biomass and an effluent are formed. The effluent is transported to an ATAD reactor and therein subjected to an aerobic fermentation, at a temperature of between 40 and 70°C. In the ATAD reactor a second biomass is formed and a supernatant effluent, which is subjected to an additional purification process for removing additional nitrogen and phosphor. The excess of second biomass is oxidised for example in the presence of hydrogen peroxide, at a pH of between 1 and 6 and a temperature of between 50 and 70°C. Thereby, the second biomass is converted into an oxidised effluent which is returned to the ATAD reactor.

The process disclosed in NL-A-9301151 however presents the disadvantage that the second biomass formed in the ATAD reactor is subjected to an oxidation, as a consequence of which the energy present in this biomass is not efficiently used.

Both above disclosed methods present the disadvantage that the biomass hardly sediments and that the hydrolysis of the AAD biomass on the one hand and the oxidation of the ATAD biomass on the other hand, are additional process steps which consume a large amount of chemicals if a complete processing of the biomass is envisaged. Moreover they involve the complete destruction of the biomass and do not provide a useful use of the energy present in the second biomass. There is thus a need to a method for the processing of sludge which provides a better use of the energy present in the sludge.

Therefore, the present invention envisages to provide a device with which not only an optimum processing of sludge may be obtained, but with which also an optimum fermentation is provided of the biomass generated with this method, so that a minimum amount of residual sludge, in particular biomass is formed.

This is achieved according to the present invention with the technical features of the first claim.

In the method of the present invention sludge is subjected to an anaerobic biological fermentation process. Subsequently, the sludge resulting form the anaerobic fermentation, i.e. liquid phase and the generated first biomass included, are subjected to an aerobic fermentation.

The method of this invention comprises the following steps:
a. the sludge is subjected to an anaerobic biologic fermentation process. As a consequence of this anaerobic fermentation, biogas is released which mainly comprises methane, an energy source suitable for further use. Mostly, also an amount of carbon dioxide is present. In the course of the anaerobic fermentation process an amount of a first sludge or biomass is formed in a first liquid phase, whereby a first significant decrease of the concentration of the solid state components in the liquid phase is realised
b. the thus formed sludge, i.e. biomass as well as interstitial liquid phase, are transported to an aerobic fermentation tank and are subjected therein to an aerobic fermentation in the presence of an oxygen containing gas, whereby the temperature in the course of the aerobic fermentation is maintained between 25 and 70°, preferably between 45 and 70°C, more preferably between 45 and 65°C. In the course of the aerobic fermentation a second sludge or biomass is formed and a second effluent, whereby a further reduction of the solid state content may be realised.
c. The content of the aerobic fermentation tank is transported to a separation device, to separate the second biomass from the purified effluent.

The method of this invention is suitable for the processing of both primary and secondary organic sludge.

By first subjecting the sludge to an anaerobic fermentation, it is achieved that the organic waste present in the sludge is fully and as such available for anaerobic fermentation, as a consequence of which methane production in the anaerobic fermentation may be maximised. In the aerobic fermentation following the anaerobic fermentation, the remaining waste which could not be processed in the anaerobic fermentation as well as the first anaerobic biomass, are processed. The at least partly processing of the anaerobic biomass in the aerobic fermentation allows reducing the consumption of chemicals in the subsequent hydrolysis.

The inventor has surprisingly found that the aerobic biomass is capable of fermenting the biomass formed in the anaerobic fermentation and not exclusively the interstitial liquid, at a minimum risk to nitrification, in particular in case the temperature is maintained above 45°C. The inventor has further found that the aerobic biomass shows a much faster cultivation and growth as compared to the anaerobic biomass and that the anaerobic fermentation has a relatively longer processing time as compared to the aerobic fermentation. This has the consequence that the anaerobic fermentation tank and the anaerobic biomass mostly occupy a much larger volume as compared to the aerobic fermentation tank and aerobic biomass. The finding that the aerobic biomass is capable of fermenting the anaerobic biomass, allows a fast fermentation and processing of a possible excess of anaerobic biomass and to maintain the volume of anaerobic biomass within acceptable margins, without necessitating an intermediate process step, such as oxidation or hydrolysis.

In the process of this invention thus not only the effluent generated in the anaerobic fermentation is subjected to an aerobic fermentation as is known from the state of the art, but also the biomass generated in the anaerobic fermentation. The present invention thus provides the possibility to simultaneously process the sludge and/or solid state components of organic nature present in the waste water and to accomplish a purification of the interstitial water present in the sludge. Thereby the present invention simultaneously provides the possibility to control the amount of biomass formed and present in the method of this invention, by using the biologic processing of one biomass by the other, at a minimum consumption of chemicals.

Preferably the second biomass originating from the aerobic fermentation tank is subjected to a physical disintegration, following which the aerobic biomass is rendered suitable for direct introduction in the anaerobic fermentation. In the disintegration process, the biomass which usually takes the shape of flocks of associated micro-organisms is disintegrated into individual cells or smaller groups of cells. The disintegration also involves a mechanical attack of the cell walls so that the cell contents is released and is suitable for further processing in the anaerobic fermentation.

Before conducting the separated second biomass to the disintegrator, it is preferably subjected to a chemical and/or thermal hydrolysis for weakening the cell walls of the micro-organisms present in the biomass, so as to obtain an improved decomposition in the disintegrator. The material originating form the chemical and/or thermal hydrolysis is preferably recycled to one or more of the disintegrator, the acidogneous, the anaerobic or aerobic fermentation tank, since the hydrolysed material contains nutrients for the acidogneous, the anaerobic or aerobic biomass.

In the present invention, the anaerobic fermentation may be preceded by an acidogenic fermentation, depending on the nature of the sludge. Carrying out the acidogenic fermentation is mainly important when treating primary sludge, which contains fastly acidifying components, for example starch. In the acidogenic fermentation, the supplied sludge, and preferably also the biomass originating from the aerobic fermentation is subjected to a biologic hydrolysis in slightly acid medium, by the action of micro-organisms and the enzymes produced by them, with the aim of at least partly hydrolysing the sludge. The hydrolysis causes a conversion of the organic material into its components, for example volatile fatty acids, amino acids, etc. The inventor has now found that these building blocks constitute an optimum substrate fro the methageneous micro-organisms present in the anaerobic first biomass, as a consequence of which anaerobic fermentation is promoted and an improved conversion of the sludge is realised.

The method of this invention presents the particular advantage that it is suitable for the processing of organic sludge and/or solid state components of organic nature present in waste water, with a high content of dissolved or non-dissolved organic components. With a high content of organic components is meant a content of between approximately 20.000 and 200.000 mg COD (chemical oxygen demand) per liter. The content of organic components may however be higher or lower.

This invention also relates to a device for the degradation of organic sludge and of solid components of organic origin present in waste water and for the processing of organic sludge, as disclosed in the accompanying claims.

The device of the present invention for the degradation of organic sludge and of solid components of organic origin present in waste water comprises a supply for supplying the sludge and/or solid components of organic origin present in waste water to an anaerobic fermentation tank to at least partly fermenting the sludge and/or organic components to biogas and a first biomass in a first liquid phase, a connection for transporting the first biomass, remaining sludge and first liquid phase to an anaerobic fermentation tank to subject the first biomass to an aerobic fermentation into carbon dioxide, second interstitial effluent and a second biomass and a connection for transporting the second biomass and second interstitial effluent from the aerobic fermentation tank to a separation device for separating solid components and effluent and a connection for returning the solid components to one or more of the anaerobic and aerobic fermentation tank.

The device of the present invention preferably comprises at a position between the supply and the aerobic fermentation tank a disintegrator to physically disintegrate the solid components originating from the separation device.

Another preferred embodiment of this invention is characterised in that the device comprises between the disintegrator and the anaerobic fermentation tank, an acidogenic fermentation tank to subject the supplied organic material to a microbiologic hydrolysis.

An additional preferred embodiment of the device of this invention is characterised in that the separation device is connected to a chemical reactor for hydrolyising the solid components, in which the chemical reactor is connected to one or more of the disintegrator, the acidogenic reactor, the anaerobic fermentation tank and the aerobic fermentation tank for returning the hydrolysed solid components to one or more of them.

The invention is further elucidated in the enclosed figures and figure description.

Figure 1 shows a schematic setting of the basic units of the device of this invention.

Figure 2-4 show a schematic setting of the three preferred embodiments of the device of this invention.

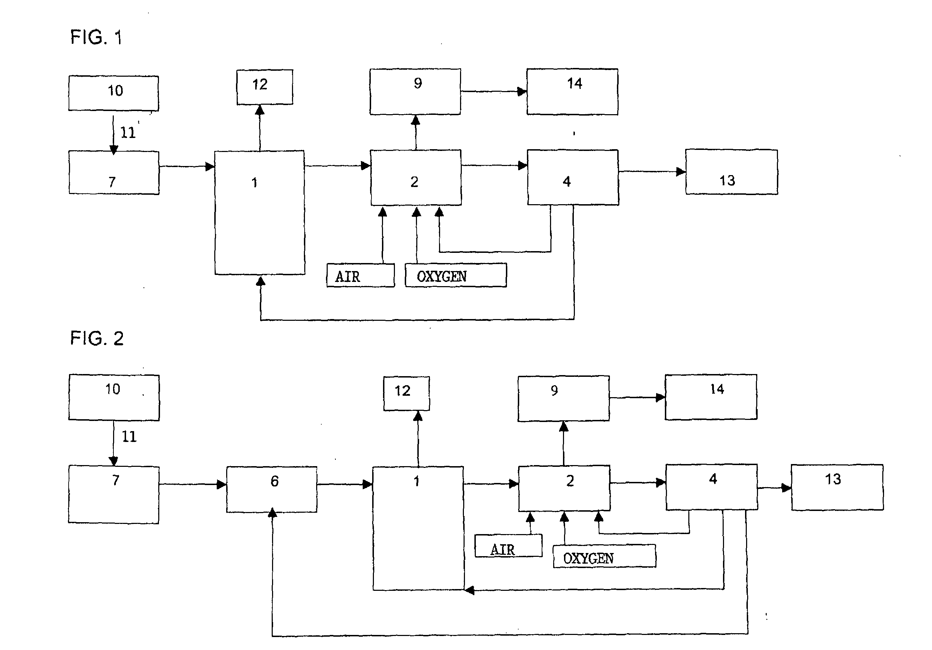

The device shown in figure 1-4 comprises a supply 11 for the sludge 10 or components of organic nature present in waste water to be processed, a mixing device 7 for homogenising the supplied sludge, a fermentation tank for the anaerobic fermentation 1 of the sludge to a first biomass and a first effluent and a fermentation tank for carrying out the aerobic fermentation 2 of the first biomass and first effluent originating from the anaerobic fermentation into a second biomass and second effluent.

The presence of a homogenising device 7 is preferred to provide for an optimum processing of primary sludges with varying composition. Primary sludge for example mainly contains organic remainders and diverging types of waste, such as for example organic raw materials, food remainders etc. To guarantee optimum sludge processing and a maximum efficiency in all process steps, it is advisable to reduce and homogenise the sludge in a homogenising device 7 in advance. Secondary sludge on the other hand which is produced in biological waste water purification devices, consists mainly of micro-organisms such as for example bacteria, fungi, yeasts, protozoa, nematodes, algues etc.

After having been homogenised the sludge is conducted to the anaerobic fermentation tank 1 and subjected to an anaerobic fermentation, in particular an anaerobic methane fermentation process, whereby biogas is generated. The biogas mainly comprises methane and carbon dioxide gas and is suitable for producing electricity and/or heat. The anaerobic fermentation is preferably carried out at a temperature of between 25-65°C. A temperature between 30 and 40°C is particularly preferred in case mainly secondary sludge is processed, which contains a high amount of proteins, so that ammonia production and thus the risk to inhibition of the anaerobic biomass may be minimised. A temperature between 50 and 60°C is preferred when processing primary sludge. The higher temperature allows a faster fermentation, whereby because of the composition of the primary sludge only a slight production of ammonia occurs. The anaerobic fermentation is preferably carried out at a pH of between 6.5 and 8.5, more preferably between 6.8 and 8.2 as within this pH range an optimum activity of the metageneous micro-organisms has been observed.

In the anaerobic fermentation the organic components are mainly converted into methane. Placing the anaerobic fermentation in a front stage presents the advantage the sludge is converted to a large extent into energetically interesting material. Only part of the remaining needs a further aerobic processing, so that a smaller amount of oxygen is needed to obtain a maximum conversion in the aerobic fermentation as compared to the situation where the organic components in the sludge are as such fermented in aerobic circumstances. With the method of the present invention thus oxygen consumption in the aerobic fermentation may be minimised.

From the state of the art of the purification of waste water it is known to have the aerobic fermentation upfront in the process. This provides for a natural heating of the reaction mixture because of the exothermal character of the aerobic fermentation and allows to minimise heat supply. Putting the aerobic fermentation upfront of the anaerobic fermentation however presents the disadvantage that the organic components present in the sludge are mainly converted in the aerobic fermentation, as a consequence of which less organic material remains for methane production in the anaerobic fermentation.

After termination of the anaerobic fermentation, the organic material, this is the first anaerobic biomass and first liquid phase as well as any remaining sludge, are conducted to the aerobic fermentation 2 and subjected to an aerobic fermentation.

The aerobic fermentation is preferably carried out at a temperature of between 25 and 70°C, more preferably between 45 and 70°C, most preferably between 45 and 65°C. A minimum temperature of 40°C is preferred to minimise the risk to nitrification.

To effectuate aerobic fermentation an oxygen containing gas is supplied to the aerobic fermentation tank 2. In the course of the aerobic fermentation the organic material originating from the anaerobic fermentation is converted, whereby a significant amount of carbon dioxide, and to a lesser extent of ammonia, are produced. The ammonia dissolves in the reaction mixture, which involves a pH increase.

In the method of this invention in the course of the aerobic fermentation preferably oxygen is supplied. Thereby the pure oxygen supply is controlled in such a way that all oxygen is consumed. The inventor has observed that oxygen dissolves well in the reaction mixture, so that a good availability of the oxygen in the biomass is provided. To provide an oxygen solubility in the reaction mixture which is as good as possible, the oxygen is dissolved in a small amount of aerobic sludge beforehand, after which this sludge is supplied to the aerobic fermentation tank.

In the course of the aerobic fermentation carbon dioxide is formed. The inventor has observed that the majority of the carbon dioxide dissolves in the reaction mixture. This has the consequence that the pH of the reaction mixture decreases. To prevent the pH of becoming too low, it may be chosen to supply an oxygen/air mixture in stead of pure oxygen, to expel the excess of carbon dioxide gas from the reaction mixture through the air. In order to simultaneously minimise the risk to foaming because of the nitrogen present in the air, it will be attempted to minimise air supply and use an oxygen/air ratio which is as high as possible.

As has been explained above, controlling the oxygen/air mixture not only allows maintaining the pH of the reaction mixture within the desired ranges without necessitating the use of chemicals (mineral or organic acids or bases), but it is also possible to maintain foam formation within practical limits.

The aerobic fermentation is mostly carried out at a pH between 6 and 9. Depending on the nature of the sludge it may be desired to have the aerobic fermentation proceeding at a higher pH (for example between 8-9) or a lower pH (for example between 6 and 7). The pH is controlled by varying the composition of the supplied gas from pure oxygen to a mixed oxygen/air mixture.

Before being further processed or discharged, the biomass obtained from the aerobic fermentation or the sludge/water mixture obtained from the aerobic fermentation is preferably conducted to a separation device 4, for separating the second biomass and effluent 13. Thereto the devices known to the person skilled in the art may be used such as for example a centrifuge, a press, an ultra-filter or any other device known to the person skilled in the art. The effluent may for example be returned to the waste water purification device from which the sludge originates. The organic components or the solid components are preferably returned to one or more of the anaerobic fermentation tank 1, the aerobic fermentation tank 2, the acidogenic reactor 3, a disintegrator 6 or a chemical reactor 5 for hydrolysing the second biomass.

Before being returned to the process, the solid components originating from the separation device 4 are preferably conducted to a disintegrator 6 to accomplish a physical disintegration, as a consequence of which the aerobic biomass is optimised for direct introduction in the anaerobic fermentation (see figure 2). In the disintegration the biomass which mostly takes the form of flocks of associated micro-organisms, is disintegrated ito individual cells or smaller groups of cells. The disintegration also involves a mechanical attack of the cell walls so that the cell contents is released and is suitable for further processing in the acidogenic and/or anaerobic fermentation. Disintegration is mostly carried out physically, for example by cavitation or ultrasonic. Cavitation is a physical process which is induced mechanically, for example by subjecting the solid components to a sudden pressure drop, as a consequence of which the cells of solid components seem to explode and their contents is released into the liquid phase.

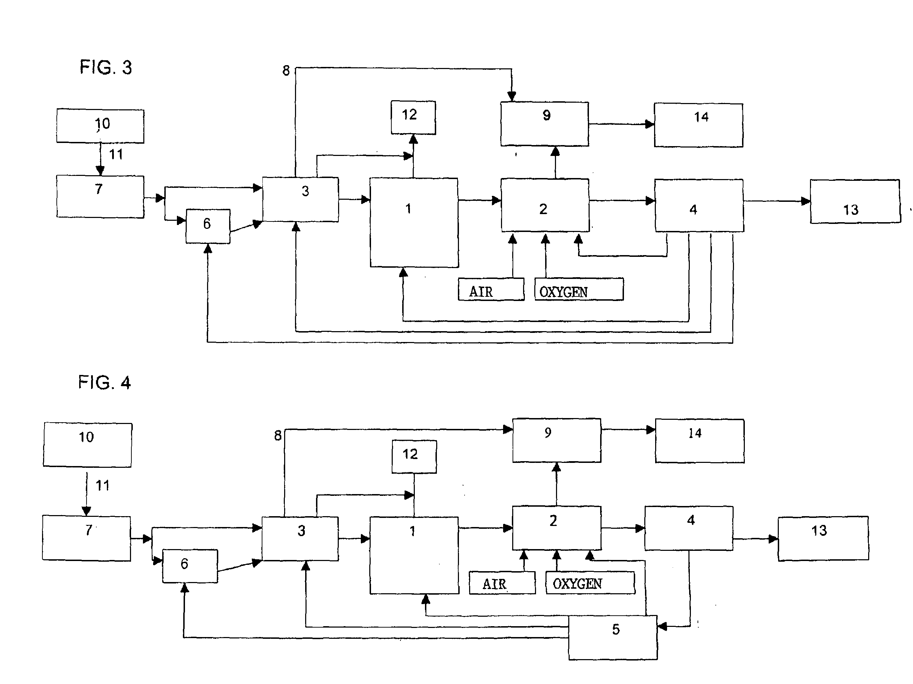

Before being returned to the process, the solid components may be subjected to a chemical hydrolysis in reactor 5, as is shown in the preferred embodiment of figure 4. In this hydrolysis the cell walls of the micro-organisms of the biomass are weakened, with the aim of facilitating their further decomposition in the anaerobic and aerobic fermentation. The hydrolysis may be caused by a thermal treatment, by subjecting the biomass for example to a temperature of 45-65°C, which is the temperature of the mixture when leaving the aerobic fermentation tank 2 and the mixing device 4. A higher temperature of 100°C may also be suitable, depending on the composition of the solid components. The hydrolysis can also be accomplished chemically. Depending on the nature of the biomass to be hydrolysed, the hydrolysis is carried out in alkaline medium at a pH between 10 and 12 for example in the presence of a mineral base or in acid medium at a pH between 1 and 3 in the presence of a mineral acid.

As is shown in the preferred embodiment of figure 3 and 4 the device of this invention may further contain an acidogenic fermentation tank 3, in which the organic material is subjected to a microbiologic acidification process under the influence of micro-organisms. In this microbiologic hydrolysis a part of the organic material is hydrolysed and converted in volatile fatty acids which form an optimum substrate for methanogeneous bacteria in the anaerobic fermentation tank 1. In the course of this acidification also acid gasses 8 are formed, mainly carbon dioxide gas and some hydrogen gas. These gasses may be discharged to a gas purification device 9 and discharged from there 14, or be discharged together with the biogas formed in the anaerobic fermentation and be processed.

The organic material originating from the chemical hydrolysis reactor 5 may, depending on the composition of the device and composition of the organic material, be returned to the disintegrator 6, the acidogenic fermentation tank 3, the anaerobic 1 or aerobic fermentation tank 2.

With the above-described method and device, it is possible
- To obtain a virtually complete fermentation of the supplied sludge. Often a fermentation of 97-98% of the floating organic solid state components present in the sludge may be accomplished;
- To obtain a maximum biogas production, i.e. 40-80 % more than with a conventional single step methane fermentation process. One of the reasons is that the sludge is not subjected to heavy chemical oxidation in none of the process steps, as a consequence of which it would no longer be available for methane production in the anaerobic fermentation. To the contrary in this invention the from the anaerobic fermentation remaining organic material is processed in the aerobic fermentation. The new aerobic biomass which is generated therein, is separated, treated and returned tot eh anaerobic fermentation tank, which results in the additional production of biogas. This invention thus provides in a virtually complete recycling of both the organic components present in the sludge and the biomass.
- To control the pH in the course of the aerobic fermentation without the need to supply chemicals (acids or basis). PH control takes place by controlling the oxygen concentration in the air supplied tot eh aerobic fermentation.
- That production of bad smelling gasses is minimised and may be further improved by subjecting the released gasses to a purification
- To minimise heat supply. The reason is that the organic material originating from the aerobic fermentation is separated off and is recycled to the anaerobic fermentation directly or indirectly. This causes heating of the mixture present in the anaerobic fermentation tank.

## Claims

1. A method for the degradation of sludge and/or solid components of organic origin present in waste water, according to which the sludge is successively subjected to an anaerobic and aerobic biologic fermentation process, **characterised in that** the method comprises the following steps
a. The sludge is subjected to an anaerobic biologic fermentation process, in which a first biomass with a first interstitial liquid phase is formed and methane gas is produced,
b. The thus obtained first biomass and first interstitial liquid phase are transported to an aerobic fermentation tank and subjected to an aerobic fermentation in the presence of an oxygen containing gas, during which the temperature is maintained between 25 and 70°C and a second biomass with an interstitial second effluent is formed,
c. After which the thus formed second biomass and interstitial effluent are conducted towards a separator for separating the sludge into purified effluent and a second biomass.

2. A method as claimed in claim 1, **characterised in that** the aerobic fermentation is carried out at a temperature between 45 and 70°C, preferably between 45 and 65°C.

3. A method as claimed in claim 1 or 2, **characterised in that** in the course of the anaerobic fermentation process the temperature is maintained between 25 and 65°C.

4. A method as claimed in any one of claims 1-3, **characterised in that** the anaerobic biologic fermentation is carried out at a temperature of between 30 and 40°C or a temperature between 50 and 60°C.

5. A method as claimed in any one of claims 1-4, **characterised in that** the anaerobic fermentation is carried out at a pH of between 6.5 and 8.5, preferably between 6.8 and 8.2.

6. A method as claimed in any one of claims 1-5, **characterised in that** the second biomass is subjected to a physical disintegration with the aim of at least partly disintegrating the second biomass and of mechanically affecting the cell wall of the micro-organisms present in the biomass, after which the disintegrated material is conducted towards the aerobic fermentation tank.

7. A method as claimed in any one of claims 1-6, **characterised in that** after termination of the aerobic fermentation, the second biomass is subjected to a chemical and/or thermal hydrolysis and the hydrolysed mass is returned to one or more of an acidogenic fermentation tank, the anaerobic or aerobic fermentation tank.

8. A method as claimed claim 7, **characterised in that** the hydrolysis is carried out at a pH of between 10 and 12.

9. A method as claimed in cliam 7, **characterised in that** the hydrolysis is carried out at a pH of between 1 and 3.

10. A method as claimed in any one of claims 7-9, **characterised in that** the hydrolysis is carried out at a temperature of between 35 and 150°C, preferably between 45 and 65°C.

11. A method as claimed in any one of claims 1-10, **characterised in that** the anaerobic fermentation is preceded by a step in which the sludge is subjected to an acidogenic fermentation to at least partly biologically hydrolyse the sludge.

12. A method as claimed in any one of claims 7-11, **characterised in that** the hydrolysed second biomass is conducted to and subjected to one or more of the acidogenic fermentation, the anaerobic fermentation and the aerobic fermentation.

13. A method as claimed in any one of claims 1-12, **characterised in that** in the course of the aerobic fermentation oxygen, air or a mixture of both are introduced into the aerobic fermentation tank, in such an amount that all added oxygen is consumed in the aerobic fermentation.

14. A device for the degradation of sludge and/or solid components of organic origin present in waste water, comprising a supply (11) for supplying of the sludge and/or solid components of organic origin present in waste water (10) to an anaerobic fermentation tank (1) to at least partly fermenting the sludge and/or organic components to biogas and a first biomass in a first liquid phase, a connection for transporting the first biomass, remaining sludge and first liquid phase to an aerobic fermentation tank (2) to subject the first biomass to an aerobic fermentation until carbon dioxide is formed, second interstitial effluent and a second biomass, whereby the aerobic fermentation tank contains means for maintaining the temperature between 25 and 70°C, preferably between 45 and 70°C, and a connection for transporting the second biomass and second interstitial effluent from the aerobic fermentation tank (2) to a separation device (4) for separating solid components and effluent (13) and a connection for returning the solid components to one or more of the anaerobic (1) and aerobic (2) fermentation tank.

15. A device as claimed in claim 14, **characterised in that** the device comprises at a position between the supply (11) and the anaerobic fermentation tank (1) a disintegrator (6) to subject the solid components originating from the separation device (4) to a physical degradation.

16. A device as claimed in claim 15, **characterised in that** the device comprises between the disintegrator (6) and the anaerobic fermentation tank (1), an acidogenic fermentation tank (3) to subject the supplied organic material to a microbiologic hydrolysis.

17. A device as claimed in claim 16, **characterised in that** the separation device (4) is connected to a chemical reactor (5) for hydrolyising the solid components, in which the chemical reactor (5) is connected to one or more of the disintegrator (6), the acidogenic reactor (3), the anaerobic fermentation tank (1) and the aerobic fermentation tank (2) for returning the hydrolysed solid components to one or more of them.
